# EUROPEAN PATENT APPLICATION

(11) **EP 2 868 748 A1**
(43) Date of publication of application: **06.05.2015**
(21) Application number: 13191106.7
(22) Date of filing: 31.10.2013
(51) Int. Cl.: C12N 15/82, A01H 4/00

(54) **A method for protein production in doubled haploid plants**

(71) Applicant: Locusia Oy, 00890 Helsinki (FI)
(72) Inventor: Kimmo, Koivu, 00890 Helsinki (FI)
(74) Representative: Kahu, Sirje

(57) **Abstract**

A method to provide transgenic doubled haploid plants is provided. The doubled haploid plants of this disclosure express heterologous gene products in their seeds. The disclosure also provides a clonal-like isogenic production system in dicotyledonous plant seeds where transgene expression can be controlled. The disclosure specifically provides a system to produce high quality and quantity of heterologous gene product in doubled haploid *Camelina saliva* seeds.

## Description

### FIELD OF INVENTION

This invention relates to a clonal-like isogenic production system in plant seeds. More specifically this invention relates to a clonal-like isogenic production system of recombinant proteins and modified seed oils in doubled haploid dicotyledonous plant seeds.

### BACKGROUND OF THE INVENTION

The chromosomes of higher plants exist in homologous pairs, and are called diploids. Gametic cells contain half of the normal number of chromosomes and are said to be haploid. When two gametic cells unite, the two sets of haploid chromosomes combine to create a set of diploid chromosomes. A doubled haploid plant is formed when the chromosomes of a haploid cell are doubled such that each chromosome in a homologous pair is identical.

Doubled haploid -methodology is commonly used in agronomically important crops, such as corn, to speed the development of new cultivars. With traditional methods it takes about 10 years to develop a new cultivar, while with doubled haploid -technology the breeding period can be reduced to about third. Haploid plants are commonly produced using one of four methods: a) culture of anthers or microspores (androgenesis), b) culture of unfertilized ovules (gynogenesis), c) interspecific or intergeneric crosses followed by chromosome elimination, and d) pollination with irradiated pollen. In many species the culture of isolated microspores or anthers is the most frequently used method of haploid production.

Genetic engineering is an essential part of plant breeding today. Haploid systems are commonly used for microspore mutagenesis. The haploid system is well suited for genetic transformation as well. For example US2008/0216198 provides a method to transform haploid embryos of *Zea mays* and then treat the transformed embryo cells with chromosome doubling agent. US2009/0293141 discloses *Agrobacterium* -transformation of immature embryos of a double hybrid corn line.

There are also examples of transgenic plants produced by transforming microspores or microspore originated callus and then cultivating the transgenic haploid plants. For example US 7,297,838 discloses creating an anther culture of flax -plants (*Linum usitatissimum*) and transforming the anther-culture obtained callus with *Agrobacterium* harboring beta-glucuronidase encoding *gus*-gene.

However, the examples of transforming double haploid plants are scarce. Tsukazaki et al. 2002 discloses transformation of hypocotyls of doubled haploid *Brassica oleracea* plants. The hypocotyls were transformed with beta glucuronidase encoding *gus*-gene and the gene expression was tested of the hypocotyl or leaf segments. The best transformation rate disclosed in this publication was 3.1% (3 transformants per 98 explants).

Kazan et al. 1997 disclose *Agrobacterium* -mediated transformation of doubled haploid *Brassica napus* lines. The transformation was conducted with hypocotyl segments and roots as explants. The *Agrobacterium* strains harbored vectors containing GUS encoding sequences and GUS-expression was detected in petals, leaves, stamens and anthers of the transformed doubled haploid lines. Similarly as in Tsukazaki the transformation rates were extremely low and in many conditions no transformants were obtained.

Cross pollination rates of *Brassica napus* can be 20-30%, which would create difficulties to maintain a doubled haploid line from generation to generation. Therefore, the doubled haploid Brassica lines of Tsukazaki et al and Kazan et al may be suitable mainly for research purposes as new lines would need to be generated at time to time.

Accordingly, there are methods to produce doubled haploid lines from a number of plant species. However, the critical step is managing to produce the microspore cultivation and the conditions are highly dependent on the plant species. There are a number of factors affecting microspore embryogenesis including genotype, donor plant growth conditions, stage of microspore development, composition of the culture medium, and environmental conditions during culture. The frequency of embryo production will depend on whether or not these conditions are optimal and varies depending on the species. Furthermore, in order to maintain the doubled haploid mother lines the species has to be selected with the cross pollination frequency in mind.

Once the microspore cultivation is established for a species, it is relatively common to use the microspore originated cells or embryos for mutagenesis or genetic transformation. However, it is not a common practice to grow double haploid plants and use these plants as explants for genetic transformation. This approach has been taken by Tzukazaki et al. and by Kazan et al. to transform *Brassica* double haploid plants with GUS-gene expressing in plant shoots. However, they were able to obtain only extremely low transformation rates.

Ferrie and Bethune 2011 have published a protocol for microspore embryogenesis for *Camelina sativa.* However, there is no record of anyone having used doubled haploid *Camelina sativa* plants as explants for gene transformation. Neither do we have any record of anyone having transformed double haploid dicotyledonous plants to express heterologous proteins in their seeds.

There is a constant need for a system to produce large quantities of uniform quality of heterologous proteins and other recombinant gene products for industrial and pharmaceutical uses.

Additionally there is a need for a production system where the expression level of heterologous products could be controlled.

This invention will provide solutions to these needs and others not provided by currently known art.

### SUMMARY

It is an object of this invention to provide doubled haploid lines of *Camelina sativa* plants.

It is another object of this invention to provide a transformation system for doubled haploid *Camelina sativa* plants.

It is yet another object of this invention to provide a method to introduce seed expressing heterologous genes into doubled haploid dicotyledonous plants.

Yet another object of this invention is to provide a method to produce heterologous proteins in doubled haploid dicotyledonous plant seeds for industrial and pharmacological uses.

It is an object of this invention to provide a clonal-like isogenic production system in dicotyledonous plant seeds to produce heterologous proteins and modified seed oils.

It is still another object of this invention to provide a clonal-like isogenic production system in dicotyledonous plant seeds to produce heterologous proteins in large quantities with high uniformity of the product.

It is another object of this invention to provide a clonal-like isogenic production system in dicotyledonous plant seeds to produce altered seed oils in large quantities with high uniformity of the product.

It is an object of this invention to provide a clonal-like isogenic production system where the expression level of heterologous gene product can be controlled by manipulating the copy number of the transgenes in doubled haploid plant material.

A further object of this invention is to provide a stable double haploid clonal-like isogenic *Camelina sativa* mother seed line, the seeds of which are germinated and used as an explant source for *Agrobacterium* mediated transformations.

Still another object of this invention is to provide a stable double haploid clonal-like isogenic *Camelina sativa* mother seed line, the seeds of which are germinated and grown to mature flowering plants that are used for *Agrobacterium* mediated flower dip transformations.

A further object of this invention is to provide a transgenic double haploid seed of *Camelina sativa* plants.

It is an object of this invention to provide a method to produce one or more heterologous gene products in dicotyledonous plant seeds, said method comprising the steps of:
a) providing a haploid cell culture;
b) obtaining haploid embryos or callus from the a haploid cell culture;
c) regenerating the embryos or the callus to double haploid plantlets with shoots and roots;
d) obtaining double haploid plants from the doubled haploid plantlets;
e) obtaining double haploid seeds from the mature double haploid plants;
f) surface sterilizing the double haploid seeds;
g) germinating the seed of step f) *in vitro* to form doubled haploid plantlets or sprouts;
h) transforming explants from the double haploid plantlets or sprouts with *Agrobacterium tumefaciens* carrying at least one vector comprising a nucleotide sequence encoding the heterologous gene product under a seed specific promoter;
i) regenerating the transformed explants to transgenic doubled haploid plants; and
j) allowing the plants to produce seeds expressing the heterologous gene product.

According to preferred embodiment the plant species may be *Camelina sativa,* the seed specific promoter may be napin promoter and the heterologous gene product may be selected from the group consisting of lauric acid-acyl carrier protein, hydrophobin, tropoelastin, an antibody, and a growth factor.

According to a preferred embodiment the tropoelastin is encoded by a synthetic gene having nucleotide sequence according to SEQ ID NO: 5.

It is another object of this invention to provide a clonal-like isogenic production system in dicotyledonous plant seeds to produce heterologous gene products in large quantities with high uniformity of the product, said system comprising:
a) a haploid cell culture to provide haploid callus or embryos;
b) a protocol to regenerate the haploid callus or embryos to doubled haploid plantlets;
c) a protocol to grow doubled haploid plants from the plantlets of step b);
d) a protocol to produce double haploid seeds of plants of step c) ;
e) a protocol to produce explants from the double haploid seeds of step d)
f) a protocol to transform a nucleotide sequence encoding the heterologous gene product under a seed specific promoter to the explants of step e);
g) a protocol to regenerate the explants to doubled haploid transgenic plants;
h) a protocol to grow the doubled haploid transgenic plants to maturity, and to collect doubled haploid transgenic seeds of the mature plants; and
g) a protocol to harvest the heterologous gene products from the transgenic seeds.

According to one preferred embodiment the clonal-like isogenic production system is provided in *Camelina sativa* seeds.

It is still another object of this invention to provide a method to control production of heterologous gene products in dicotyledonous plant seeds, said method comprising the steps of:
a) providing a haploid cell culture;
b) obtaining haploid embryos or callus from the haploid cell culture;
c) regenerating the embryos or the callus to double haploid plantlets with shoots and roots;
d) obtaining double haploid plants
e) obtaining double haploid seeds from the double haploid plants
f) surface sterilizing the seed of step e)
g) germinating the seeds of step f) *in vitro*
h) transforming explants from double haploid plantlets or sprouts with *Agrobacterium tumefaciens* carrying at least one vector comprising a nucleotide sequence encoding a nucleotide sequence encoding the heterologous gene product under a seed specific promoter;
i) regenerating the transformed explants to transgenic doubled haploid plants;
j) determining copy number of the nucleotide sequence encoding the heterologous gene product in several doubled haploid plants;
k) selecting lines with preferred copy numbers, and
l) allowing the selected lines to produce flowers and crossing the lines, thereby receiving next generation with a increased copy number and desired level of production of the heterologic gene product.

It is still another object of this invention to provide a transgenic doubled haploid seed of *Camelina sativa* expressing a heterologous gene product.

According to preferred embodiments the heterologous gene product may be expressed under napin promoter.

According to preferred embodiment the plant species may be *Camelina sativa,* the seed specific promoter may be napin promoter and the heterologous gene product may be selected from the group consisting of lauric acid-acyl carrier protein, hydrophobin, tropoelastin, an antibody, and a growth factor.

### SHORT DESCRIPTION OF THE DRAWINGS

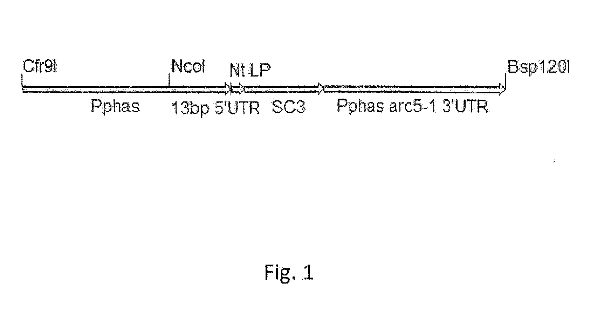

**Fig. 1** Expression cassette for seed specific production for *Shitzophyllum commune* hydrophobin SC3, constructed by PCR overlap.

### DETAILED DESCRIPTION OF THE INVENTION

### Definitions

**Doubled haploid** means a plant or plant tissue generated from a haploid cell, where each cell has two identical sets of haploid chromosomes.

**Hydrophobin proteins** means monomeric or multimeric hydrophobin proteins.

**Stably transformed plant** means a transgenic plant where the transgenic material is inserted into the genome of the plant, whereby the transgenic character is inherited according to Mendelian rules.

**A protocol to regenerate the haploid callus or embryos to doubled haploid plants** means methods as described in Example 4 for *Camelina sativa* plants. The protocol described in Example 4 may be modified for other plant species and such modifications are also encompassed by the term 'protocol to regenerate the haploid callus or embryos to doubled haploid plants'.

**A protocol to transform a nucleotide sequence encoding the heterologous gene product under a seed specific promoter to explants obtained from the doubled haploid plants** means a transformation method of a nucleotide sequence encoding a heterologous gene product under a seed specific promoter to explants as is described in Example 6 for *Camelina sativa.* The transformation is preferably *Agrobacterium* mediated transformation but other transformation methods may also be used.

**A protocol to produce explants from the double haploid seeds** means methods as described in Example 6 for *Camelina sativa* plants. The protocol described in example 6 may be modified for other plant species and such modifications are also encompassed by this term.

**A protocol to regenerate the explants to doubled haploid transgenic plants** means methods to regenerate the transgenic explants to doubled haploid transgenic plants as described in Example 6 for *Camelina sativa.* The method may be modified for other plant species and such modifications are encompassed by the term "a protocol to regenerate the explants to doubled haploid transgenic plants".

**A protocol to grow the doubled haploid transgenic plants to maturity, and to collect doubled haploid transgenic seeds of the mature plants** means methods such as described in Example 10 for *Camelina sativa* to grow the plants, to let them form inflorescence and produce seeds and finally collect the seeds from the plants. The plants may be grown in growth chambers, green house or in field conditions in a controlled manner.

**A protocol to harvest the heterologous gene products from the transgenic seeds** means method such as described in Examples 7 and 8. The protocol may vary depending on the gene product and such variations are encompassed by the term.

**Copy number** means the number of heterologous DNA inserts in a genome.

**Haploid cell culture** means an anther culture, pollen culture, microspore culture, or an ovule culture.

**Anther Culture:** Anther culture is technique by which the developing anthers at a precise and critical stage are excised aseptically from unopened flower bud and are cultured on a nutrient medium where the microspore within the cultured anther develop into callus tissue or embryoids that give rise to haploid plantlets either through organogenesis or embryogenesis.

**Pollen or Microspore Culture:** Pollen or microspore culture is an in vitro technique by which the pollen grains, preferably at the uninucleated stage, are squeezed out aseptically from the intact anther and then cultured on nutrient medium where the microscope, without producing male gametes, develop into haploid embryoids or callus tissues that give rise to haploid plantlets by embryogenesis or organogenesis.

**Ovule Culture:** Ovule culture is an experimental system by which ovules are aseptically isolated from the ovary and are grown aseptically on chemically defined nutrient medium under controlled conditions.

**Allopolyploidy** is a common genetic status of several plants: the plant has more than two sets of diploid chromosomes in its genome.

*Camelina sativa* is an allohexaploidic species and it has actually three sets of diploid chromosomes originating from ancient crossings of three relative species. The allohexaploid nature of the genome of *Camelina sativa* may have multiple implications. Polyploids are generally thought to be more adaptable to changing environments and more flexible in occupying larger niches than any one of their ancestors. On genetic level, recessive mutations in a hexaploid genome could be masked by redundant homologous loci. Allohexaploidy increases the diversity and genetic variation between generations. In transgenic allopolyploidic plants the multiple sets of diploid chromosomes will affect quantity and quality of recombinant protein. Therefore, allohexploidic *Camelina sativa* as a target for production of recombinant gene products may not provide an optimal result.

In this disclosure, it is shown how recombinant gene products can be produced in high and uniform quality in seeds of double haploid *Camelina sativa* plants.

In this disclosure a clonal production system in plants is provided. The production system is provided in doubled haploid seeds. *Camelina sativa* is a preferred species for the method disclosed in this disclosure due to its high frequency of self pollination. The same concept may be applied to several other dicotylenous plant species, such as *Nicotiana tabacum, Brassica* sp., *Medigago sativa,* sunflower, safflower, soybean and alfalfa. However, these plant species may require generation and selection of new mother lines due to cross pollination as opposed to primarily self pollinating *Camelina.*

Generally the production system in doubled haploid seeds can be established to produce heterologous proteins for industrial and pharmaceutical uses. Examples of industrial proteins to be produced in the clonal production system of this disclosure include but are not limited to hydrophobins, and tropoelastins. Examples of pharmaceutical proteins to be produced in the clonal production system of this disclosure include but are not limited to growth factors and recombinant antibodies. The clonal-like isogenic production system of this invention is also suitable to produce modified seed oils for human nutrition or industrial purposes. An examples of modified oils for human nutrition is long chained polyunsaturated fatty acids.

Specifically this disclosure provides constructs to express heterologous gene products in seeds. Examples of plant promoters and terminators directing the expression into the seeds, are napin -promoter and -terminator.

According to a preferred embodiment of this invention a doubled haploid line of *Camelina sativa* plant regenerated from microspore culture. The doubled haploid plants are grown in green house and surface sterilized seeds of the plants are germinated *in vitro* and used as explants for *Agrobacterium* transformation. The transformed doubled haploid explants are regenerated to shoots and roots and transferred to green house conditions. The transgenic doubled haploid plants are let to bloom and self pollinate and the transgenic seeds are used for extraction of the recombinant gene product.

According to one preferred embodiment the doubled haploid plants are transformed with *Agrobacterium* harboring a transformation vector with *Scitzophyllum communune* SC3 hydrophobin encoding sequences under seed specific promoter.

According to another preferred embodiment the doubled haploid plants are transformed with *Agrobacterium* harboring a transformation vector with human tropoelasting endocing sequences under seed specific promoter.

According to yet another preferred embodiment the doubled haploid plants are transformed with *Agrobacterium* harboring a transformation vector harboring California bay laureate carrier protein under a seed specific promoter.

Hydrophopins are small surface-active multipurpose proteins that are used in cell culture, nanotechnology, pharmacy, cosmetics and food production. The current art knows production of monomeric hydrophobin in *E.coli.* Also transient expression of hydrophobin as part of a fusion protein has been described (Joensuu et al. 2010). A method to stably transform pant tissue to express hydrophobin has been disclosed by the inventor if this disclosure in US 2013/0219559. However, transformation of and expression in double haploid plant material with monomeric or multimeric hydrophobins has never been shown.

Tropoelastin is used for cell culture scaffolds, artificial vein and skin crafts, cosmetics anmong a variety of other purposes. The current art knows expression of human tropoelastin in E. coli transformation system. The full natural tropoelastin protein sequence has not been introduced into the plant expression systems and specifically never to doubled haploid plants or doubled haploid seeds.

Need for recombinant antibodies and their derivatives increases continuously in diverse applications. There are various disclosures showing expression of recombinant antibodies is plant tissue, such as tobacco leaves, but doubled haploid plants have never been transformed with antibody encoding genes.

The invention is now described by means of nonlimiting examples. A skilled artisan would recognize that various modifications to the examples may be made without diverting from the spirit of the invention.

### Example 1: Cloning the expression vector for production of hydrophobin in the seeds of Double Haploid Camelina sativa

Seed specific expression cassette with *Phaseolus vulgaris* phaseolin promoter sequence, arcelin 5' UTR and arcelin 3' UTR was constructed as follows.

Genomic DNA for PCR amplification of *Phaseolus vulgaris* control elements is extracted from germinated seedlings with standard extraction methods. Alternatively, a kit such as Fermentas GeneJet Genomic DNA extraction kit is used. *Phaseolus vulgaris* phaseolin promoter sequence (Genebank accession number J01263; **SEQ ID NO:1)** is amplified with primers o1L **(SEQ ID NO:10)** and o13 **(SEQ ID NO:11)** from genomic DNA of *Phaseolus vulgaris* to correspond nucleotides 1 to 1470 of the promoter sequence. Short homology stretches with vector backbone containing *Cfr9I* restriction site at the 5'end and with *Phaseolus vulgaris* arcelin 5' UTR and expressed gene of interest at the 3' end are introduced into the sequence with PCR primers. Synthetic gene sequence with *Nicotiana tabacum* leader peptide and *Scizophyllum commune* SC3 (**SEQ ID NO: 2** for synthetic gene, **SEQ ID NO: 3** for amino acid sequence) is amplified with primers o11 **(SEQ ID NO:24)** and o12 **(SEQ ID NO:25)** simultaneously introducing short overlaps with amplified *Phaseolus vulgaris* arcelin 5'UTR sequence **(SEQ ID NO:26)** and arcelin 3'UTR sequence (Genebank accession number Z50202 to correspond nucleotides 2621-3900, **(SEQ ID NO:4).** Arcelin 3'UTR is bought as synthetic DNA and amplified with primers o5 **(SEQ ID NO:12)** and o6L **(SEQ ID NO:13),** simultaneously introducing short overlap with vector backbone, containing *Bsp120I* restriction site. All PCR reactions are constructed according to standard protocols. High Fidelity Phusion polymerase (Thermo Lab system) is used for the amplification. PCR products are combined to seed specific expression cassette by separate overlap PCR reaction and cloned into plant expression vector with *Cfr9I*/*Bsp120I.*

### Example 2: Cloning the expression vector for production of tropoelastin in the seeds of Double Haploid Camelina sativa

Synthetic sequence for part of *Arabidopsis thaliana* seed storage albumin 2 signal peptide (2S2 SP) coded by nucleotides 80 - 118 of Genebank accession number NM_118849 and human tropoelastin (ELN) coding for amino acid sequence coded by nucleotides 170 - 2167 of Genebank accession number NM_001081753 (**SEQ ID NO: 5** for synthetic gene, **SEQ ID NO:6** for amino acid sequence) is obtained from GeneScript. The 2S2-ELN is amplified by PCR from GeneScripts vector pUC57 using primers o115 **(SEQ ID NO: 14)** and o117 **(SEQ ID NO:15).** 6xHis-KDEL-Stop-fragment is made by PCR using two overlapping primers o116 **(SEQ ID NO:16)** and o118 **(SEQ ID NO:17).** 2S2-ELN and 6xHis-KDEL-Stop-fragment have homologous overlap and are joined together in second PCR using primers o115 **(SEQ ID NO:14)** and o118 **(SEQ ID NO:17).** In addition to homology to ELN, 6xHis-KDEL-Stop-fragment contain homology to arcelin 3'UTR.

Plant expression vector pA14-PPhas-2S2-Tarc-2-3 contain *Phaseolus vulgaris* phaseolin promoter **(SEQ ID NO:1),** *Arabidopsis thaliana* seed storage albumin 2 signal peptide (2S2 SP) **(SEQ ID NO:10)** and arcelin 3'UTR **(SEQ ID NO:4).** pA14-PPhas-2S2-Tarc-2-3 is cut with restriction enzymes XhoI and PauI between the 2S2 SP and arcselin 3'UTR and joined via end-homologies with 2S2-ELN-6xHis-KDEL-Stop fragment using Gibson Assembly reaction mix of New England Biolabs.

### Example 3: Cloning of the expression vector targeting to metabolic engineering of Double Haploid Camelina sativa

### Agrobacterium mediated transformation with lauric acid-acyl carrier protein (ACP) (EC 3.1.2.21-dodecanoyl-(acyl-carrier-protein)hydrolase) from California bay (Umbellularia californica)

Seed specific expression cassette with *Brassica napus* napin-A promoter sequence, with UcFatB1: lauric acid-acyl carrier protein (ACP) (EC 3.1.2.21-dodecanoyl-(acyl-carrier-protein)hydrolase) from California bay plant (*Umbellularia californica)* and UcFatB1 3' UTR was constructed as follows: Genomic DNA for PCR amplification of *Brassica napus* napin-A promoter sequence is extracted from germinated seedlings with standard extraction methods. Alternatively, a kit such as Fermentas GeneJet Genomic DNA extraction kit is used. *Brassica napus* napin-A promoter sequence (Genebank accession number J02798; **SEQ ID NO:7**) is amplified with primers P-Bn-napA-F **(SEQ** ID **NO-18)** and P-Bn-napA-R **(SEQ ID NO:19)** from genomic DNA of *Brassica napus*) to correspond nucleotides I to 1145 of the J02798 sequence. The 5'end of the forward primer contain XmaI restriction site. The 5'end of the reverse primer contain XmaI and BpiI restriction sites. The product is digested with XmaI and cloned into pGem7 vector, which is cut with XmaI restriction enzyme and dephosphorylated with CIP to make pGem7-P-Bn-napA. A synthetic construct containing UcFatB1 to correspond nucleotides 145 to 1293 of Genebank accession number M94159 sequence and UcFatB1 3'UTR to correspond nucleotides 1294 to 1561 of Genebank accession number M94159 sequence and flanking restriction sites for BpiI and Bsp120I **(SEQ ID NO:8)** is obtained from synthetic DNA supplier. The UcFatB1-3'UTR fragment is cut out from the suppliers vector using Bpil and Bsp120I and cloned into pGem7-P-Bn-napA which is cut with the same restriction enzymes and dephosphorylated with CIP to make pGem7-P-Bn-napA-UcFatB 1.

The expression cassette is cut from pGem7-P-Bn-napA-UcFatB1 using XmaI and Bsp120I restriction enzymes and cloned into pA5-MCS plant expression vector which is cut with same restriction enzymes and dephosphorylated with CIP to make construct pL1.

### Example 4: Generation of doubled haploid mother plant lines

Three *Camelina sativa* cultivars: Calena, Celine and Blaine Greek are subjected to microspore culture.

Donor plants are cultivated in greenhouse-like conditions, in 15 cm pots filled with peat-vermiculite mixture. Plants are watered according to need (estimated on daily basis) with tap water supplemented with mild fertilizer mixture. Soon after the inflorescences started to develop, they are removed from the plants. Small buds (∼1-3 mm) are selected to be used to set up the microspore culture.

Buds are initially briefly rinsed with 70% ethanol and subsequently rinsed with sterile, double-distilled water to completely remove the ethanol. The rinsed buds are then subjected to sterilization by incubating them for 10-15 min in 6% sodium hypochlorite, followed by several rinses (at least 3x5 min) with sterile, double distilled water.

Sterilized buds are homogenized either with a Warren blender or compatible homogenisator and suspended to ½ x Gamborg G5 medium supplemented with 13% sucrose, pH 5.8. Crude suspensions are filtered through a sterile funnel lined with a nylon screen cloth with cut-off of 41 µm. Crude microspore suspensions are then centrifuged at 130 - 150 x g for 3 min, supernatants are decanted and pellets are resuspended to fresh NLN -medium supplemented with 12.5% sucrose and 12.5% PEG4000, without glutamine, pH 5.8. Pelleting and resuspending is repeated in total of three times. The number of microspores in the medium is determined with a Burker chamber or a corresponding cell counter and the medium is adjusted to have 10,000-25,000 microspores per ml.

10 ml of microspore solution is dispensed on 100 x 15 mm Petri plates. Plates are incubated in the dark, +24°C for 72 h and supplemented with glutamine with final concentration of 0.8% w/v. Incubation in the dark is continued for 28 days. Embryos are then transferred to light, shaking at 50 rpm, with 16 h photoperiod. After about 5-10 days in the light cycle, the embryos are plated onto solid ½ x Gamborg medium supplemented with 1% sucrose, 0.8 % agar, pH 5.8. After 6-8 weeks of incubation, plantlets with well regenerated shoots and roots are transferred to a greenhouse and cultivated in peat-vermiculite mix until maturation.

Production of fertile flowers under strictly self-pollinating conditions is considered as an evidence of successful doubled haploid generation.

### Example 5. Screening doubled haploid plants for selection of suitable mother plants for transformation

Microspore cultivation is established using three different *Camelina sativa* varieties (Calena, Celine and Blaine Greek). Because different varieties respond differently in microspore cultivation two best varieties are selected based on the success of the cultivation. From both of the selected microspores cultures 100 healthy looking plantlets are chosen and the plantlets are transferred into soil and grown in a green house. The plants are grown in 16/8 hours light/dark cycle at 25/18 C_{°} degrees. The plants are watered with 0.15% 20-20-20 fertilizer as needed. Of both of the varieties 25 healthiest looking plants are selected and the chromosome number is determined in order to avoid selecting mutated or aneuploidic individuals. 10 plants of each variety, showing double haploid chromosomes are selected for further screening.

The selected 10 plants are allowed to flower, self pollinate and produce seeds. The generation thus obtained is screened first for morphological characters, including leaf area, root weight, and number of inflorescence and seeds per inflorescence. The plants are also screened by their physiological characters, such as photosynthetic efficiency, drought tolerance, seed oil content, seed protein content.

Based on these characters 5 mother plants are selected from each cultivar. The selected doubled haploid mother plants thus represent plants with highest productivity, preferably best drought tolerance and optimal capacity to be donor plants for microspore culture.

### Example 6: Transformation of doubled haploid Camelina sativa mother plant lines

The seeds of doubled haploid *Camelina sativa* mother plant are sterilized by immersing in 70% ethanol for 1 min and then treating for 5 min with Na-hypochlorite solution (2 % active Cl⁻) with an addition of Tween-20 (1 drop per 100 ml). After sterilization the seeds are washed four times in sterile water and placed on solid Murashige and Skoog (MS) agar medium (Murashige and Skoog, Physiol. Plant. 15:472-493, 1962) without sugars for germination. Sterilized seeds are germinated and grown for two weeks on solid Murashige and Skoog (MS) medium without hormones. First true leaves serve as a source of explants for transformation procedure.

*Agrobacterium tumefaciens* -strain C58 carrying plant transformation vectors as describe above are grown overnight at 28°C in liquid YEB medium (Lichtenstein and Draper, Gene Engineering of Plants. In: Glover DM (ed.) DNA Cloning-A Practical Approach, vol. 2. Oxford IRL, Oxford, pp 67-119, 1985) supplemented with 50 mg/l kanamycin and 10 mg/l rifampicin. *Agrobacterium* -culture of OD.sub.600=0.5 is used in the transformation experiments.

Leaves of *in vitro* grown doubled haploid *Camelina sativa* plants are cut with sharp knife blade on sterile wet tissue paper containing diluted overnight culture of *Agrobacterium-*solution. Leaves are cut into two halves across the leaf blade. The leaf segments with *Agrobacterium* are cultivated for 48 hours on Murashige and Skoog (MS) 0.7% agar medium supplemented with 1 mg/l 6-benzylaminopurine (BAP) and 0.3 mg/l alpha-naphthaleneacetic acid (NAA).

All the Murashige and Skoog (MS) culture media are supplemented with 1% sucrose and all *in vitro* cultures are kept at temperatures of 25°C (day) and 18°C (night) under the photoperiod of 16 h.

The explants are washed with water containing 300 mg/l ticarcillin. The explants are placed on Murashige and Skoog (MS) medium supplemented with hormones [0.7 mg/l 6-benzylaminopurine (BAP), 0.3 mg/l alpha-naphthaleneacetic acid (NAA)] and 200 mg/l ticarcillin and 0,05 mg/L imidazole. Two to three weeks old shoots are placed to the normal or half strength Murashige and Skoog (MS) medium solidified with 0.7% agar and supplemented with 200 mg/l Ticarcillin and optionally 0,1 mg/L imidazole and 0.3 mg/l alpha-naphthaleneacetic acid (NAA). Rooted shoots are transferred to soil and transgenic doubled haploid plants are grown in greenhouse or comparable conditions.

The doubled haploid plants are allowed to bloom and produce seeds. The recombinant gene expression in the transgenic doubled haploid seeds is confirmed with a northern blot analysis and the presence of the transgene is confirmed with Southern analysis.

Transformation efficiency is surprisingly high, 50-80% of explants express GUS reporter gene one week after transformation. Also the transgenic shoot regeneration efficiency is high, being 5-20% of explants. The total soluble protein of the seeds from the transformed plants varies between 0.1 and 10%. The glycosylation structure of the proteins produced in the doubled haploid seeds has more constant glycosylation structure and less variation in microheterogeneity in polypeptide sequence than proteins produced in transformed wild type plants. This is due to the minimal genetic variation in meiosis of the doubled haploid plants as the doubled haploid genome is fully homozygotic.

It is known that genomic insertion site or -position of the transgene may have major effect for transgene expression. Stable genetic background of the doubled haploid plants in subsequent generations reduces position effect because position effect is dependent on genomic background and if genetic background changes also position effect changes. Reduced position effect reduce batch -to -batch variation and makes the product more constant generation after generation.

Instead of the above described transformation method, the doubled haploid mother plants may be transformed by flower dipping method as described in US patent application number 12/933,827.According to this method Agrobacterium-mediated procedure comprises dipping flower clusters of doubled haploid *Camelina sativa* plants into a solution having an *Agrobacterium* comprising the heterologous gene sequence under the seed specific promoter as described above, a sugar and a nonionic surfactant. The method may include more than one dipping cycle, wherein a dipping cycle includes at least one step where the plant is dipped into the solution. As an alternative, the methods of the present invention can also comprise spraying the dipping solution onto the floral clusters of the doubled haploid plants or onto the sprouted seeds of the doubled haploid plants.

### Example 7. Extraction of hydrophobin from the double haploid Camelina sativa seeds

0.5 g of freshly harvested doubled haploid *Camelina sativa* seeds expressing hydrophobin were put into mortar and covered with liquid nitrogen. Seeds were ground to fine powder. Mortar was set on ice and the powder was let to let to thaw and 15 ml PBS (Phosphate-buffered saline extraction buffer: 137 mM CaCl2, 2.7mM KCl, 8.1 mM Na2HPO4 and 1.8 mM KH2PO4; pH 7) was added. Extract was transferred to test tube and centrifuged 5 minutes at 20 000 at 4 °C. The supernatant was collected into a new tube.

400 mg of triton X 114 was weighed into the 15 mil test tube. 10 ml of seed extract was preheated in 24 °C water bath for 5 min and seed extract/ Triton X114 mixture was vortexed thoroughly for 1 min. The tube was set back to 24 °C water bath for 20 minutes to separate the phases.

The lower phase containing hydrophobic was transferred by pipetting, starting from the bottom of the tube, to a new tube. 4 ml isobutanol was added to hydrophobin containing lower phase and mixed well. The tube was set to a 24 °C water bath to separate phase. The lower phase containing the hydrophobin was collected to a new test tube. Isobutanol was removed by filtration though Biogel P-6 column (Bio-Rad). Hydrophobin containing solution was stored at 4° C temperature. SDS- PAGE analysis was further used to analyze the hydrophobin contents of the solution. The total hydrophobin content of the seeds varies between 0.1 and 10%.

### Example 8 Extraction of tropoelastin from doubled haploid transgenic Camelina sativa seeds

One gram of tropoelastin expressing seeds of *C.sativa* is homogenized in liquid nitrogen and frozen powder is transferred to 50 ml test tube containing 40 ml extraction buffer containing 100 mM Tris-HCL, pH 8.0, 150 mM NaCl and 0.5% triton X-100. Extract is filtrated trough Miracloth and centrifuged 20 000 g 10 min +4°C. Supernatant is used to isolate His-taged tropoelastin using HIS-select Nickel affinity gel. Column containing 1 ml HIS-select Nickel affinity gel is equilibrated and washed using 50 mM sodium phosphate buffer, pH 8.0 with 0.3 M NaCl and 10 mM imidazole. Plant extract is applied to gravity flow column and washed with 10 ml washing buffer. Tropoelastin is eluted using two ml 50 mM sodium phosphate, pH 8.0 with 0.3 M NaCl and 250 mM imidazole. Tropoelastin content of the seeds is about 100 µg per 1 g of seeds using micro-BCA method according to manufactures (Pierce) protocol.

### Example 9. Extraction of modified oil from the transgenic doubled haploid seeds

Commercial service is used to analyze fatty acid profile and seed oil content of the transgenic doubled haploid *Camelina sativa* seeds.. Total seed oil content is comparable to that of doubled haploid mother plant and lauric acid (12:0) content is 25% and oleic acid content is degreased from 17% to 10%.

### Example 10. Upscale production system for producing recombinant proteins or modified oils in doubled haploid Camelina sativa plants

The selected mother lines are cultivated in green house like conditions and seed production of starting material for genetic transformation is thus ensured. The seeds are collected, germinated and explant material for transformation is obtained as described above. The explants are transformed with *Agrobacterium* harbouring at least one nucleotide sequence under seed specific promoter encoding a desired gene product. The transformed explants are regenerated as described above to plantlets that are transferred to green house like conditions. The production level of hydrophobin in doubled haploid *Camelina* seeds is within a range of 0.5-50 grams of hydrophobin or tropoelastin per kilogram of seeds. Accordingly a green house of 3000 square meters produces in average 2000 kg of *Camelina sativa* seeds and at a production of 25 grams per kilogram of seeds the greenhouse would produce 50 kg of hydrophobin or tropoelastin.

According to another embodiment the seeds of the doubled haploid plants are collected as above, but the seeds are transferred into sprouting facility where the seeds are allowed to sprout, the sprouted seeds are then collected, smashed and the heterologous product is collected from the smashed seed/spout mass.

### Example 11. Determination of copy number of transgenes using Southern blot analysis

T-DNA copy number is determined using Southern blot analysis. Digoxigenin-11-UTP labeled probes homologous to transgene are synthesized. Genomic DNA of the transgenic plants are digested using separately restriction enzymes, which have recognition site in T-DNA only other side of the probe sequences. Plants, which produce a single positive band using two or more restriction enzymes, are considered to carry only one copy of the T-DNA.

After crossing two single copy plants, portion of the offspring's should produce hybridizing pattern of both parents. Total genomic DNA is isolated from leaf tissue of *Camelina sativa* plants using DNeasy Plant Midi Kit according to the supplier's instructions (Qiagen). Three micrograms of DNA is digested with restriction enzymes. Digested DNA-samples are separated in a 0.7% agarose (Promega) gel overnight at 15mA current and transferred to positively charged nylon membrane (Boehringer Mannheim) using vacuum blotter. RNA probes are synthesized using T3 RNA polymerase on the pBluescript vector carrying hydrophobin gene sequence and labeled with digoxigenin-11-UTP. The membrane is hybridized and developed according to the supplier's instructions (Boehringer Mannheim, The DIG user's guide for filter hybridization). The membrane is pre-hybridized at 50°C for 2 h and hybridized at 50°C in a "DIG EasyHyb" hybridization solution (Boehringer Mannheim) overnight with a digoxigenin-UTP labelled RNA probe. The concentration of RNA probe is 100 ng/ml. After hybridization the membrane is washed in SSC buffers, blocked and detected using "Anti-Digoxigenin-AP alkaline phosphatase (Boehringer Mannheim). Chemiluminescent detection is done with CSPD-substrate and the membrane is exposed to X-ray film (Boehringer Mannheim). Presence of the transgene insertion is proved in comparison to DNA of non-transgenic Camelina sativa -plant DNA as negative control, and to plasmid DNA carrying the gene sequence mixed with non-transgenic plant DNA as positive control.

Based on the Southern blot analysis, plants with a desired copy number may be selected and crossed with each other to obtain higher copy number and accordingly a higher expression of the heterologous gene product.

### Example 12. Agrobacterium mediated transformation with mouse anti-CEA single chain fab fragment

Synthetic sequence for part of *Arabidopsis thaliana* seed storage albumin 2 signal peptide (2S2 SP) coded by nucleotides 80 - 118 of Genebank accession number NM_118849 and mouse anti-CEA single chain fab fragment (aCEA-Fv) (UniProt accession number Q921A6) (**SEQ ID NO: 20** for synthetic gene, **SEQ ID NO:21** for amino acid sequence) is obtained from GeneScript. The 2S2- aCEA-Fv is amplified by PCR from GeneScripts vector pUC57 using primers o115 **(SEQ ID NO: 14)** and aCEA-R **(SEQ ID NO: 22).** 6xHis-KDEL-Stop-fragment is made by PCR using two overlapping primers aCEA-His-F **(SEQ ID NO: 23)** and o118 **(SEQ ID NO:16).** 2S2-aCEA-Fv and 6xHis-KDEL-Stop-fragment have homologous overlap and are joined together in second PCR using primers o115 and o118. In addition to homology to aCEA-Fv, 6xHis-KDEL-Stop-fragment contain homology to arcelin 3'UTR.

Plant expression vector pA14-PPhas-2S2-Tarc-2-3 contain *Phaseolus vulgaris* phaseolin promoter **(SEQ ID NO: 1),** *Arabidopsis thaliana* seed storage albumin 2 signal peptide (2S2 SP) **(SEQ ID NO:10)** and arcelin 3'UTR **(SEQ ID NO:4).** pA14-PPhas-2S2-Tarc-2-3 is cut with restriction enzymes XhoI and PauI between the 2S2 SP and arcelin 3'UTR and joined via end-homologies with 2S2-ELN-6xHis-KDEL-Stop fragment using Gibson Assembly reaction mix of New England Biolabs.

## Claims

1. A method to produce one or more heterologous gene products in dicotyledonous plant seeds, said method comprising the steps of:
a) providing a haploid cell culture;
b) obtaining haploid embryos or callus from the haploid cell culture;
c) regenerating the embryos or the callus to double haploid plantlets with shoots and roots;
d) obtaining double haploid plants from the doubled haploid plantlets;
e) obtaining double haploid seeds from the mature double haploid plants;
f) surface sterilizing the double haploid seeds;
g) germinating the seed of step f) *in vitro* to form doubled haploid plantlets or sprouts;
h) transforming explants from the double haploid plantlets or sprouts with *Agrobacterium tumefaciens* carrying at least one vector comprising a nucleotide sequence encoding the heterologous gene product under a seed specific promoter;
i) regenerating the transformed explants to transgenic doubled haploid plants; and
j) allowing the plants to produce seeds expressing the heterologous gene product.

2. The method of claim 1, wherein the plant is *Camelina sativa.*

3. The method of claim 2, wherein the seed specific promoter is napin promoter.

4. The method of claim 1, wherein the heterologous gene product is selected from the group consisting of lauric acid-acyl carrier protein, hydrophobin, tropoelastin, an antibody, and a growth factor.

5. A clonal-like isogenic production system in dicotyledonous plant seeds to produce heterologous gene products in large quantities with high uniformity of the product, said system comprising:
a) a haploid cell culture to provide haploid callus or embryos;
b) a protocol to regenerate the haploid callus or embryos to doubled haploid plantlets;
c) a protocol to grow doubled haploid plants from the plantlets of step b);
d) a protocol to produce double haploid seeds of plants of step c) ;
e) a protocol to produce explants from the double haploid seeds of step d)
f) a protocol to transform a nucleotide sequence encoding the heterologous gene product under a seed specific promoter to the explants of step e);
g) a protocol to regenerate the explants to doubled haploid transgenic plants;
h) a protocol to grow the doubled haploid transgenic plants to maturity, and to collect doubled haploid transgenic seeds of the mature plants; and
i) a protocol to harvest the heterologous gene products from the transgenic seeds

6. The clonal-like production system of claim 5, wherein the haploid cell culture is a *Camelina sativa* microspore culture.

7. The clonal-like production system of claim 5, wherein the seed specific promoter is napin promoter.

8. The clonal-like production system of claim 7 , wherein the heterologous gene product is selected from the group consisting of lauric acid-acyl carrier protein, hydrophobin, tropoelastin, an antibody, and a growth factor.

9. The clonal-like production system of claim 7, wherein the transgenic seeds are sprouted and the gene product is collected from the sprouted seeds.

10. A method to control production of heterologous gene products in dicotyledonous plant seeds, said method comprising the steps of:
a) providing a haploid cell culture;
b) obtaining haploid embryos or callus from the haploid cell culture culture;
c) regenerating the embryos or the callus to double haploid plantlets with shoots and roots;
d) obtaining double haploid plants
e) obtaining double haploid seeds from the double haploid plants
f) surface sterilizing the seed of step e)
g) germinating the seeds of step f) *in vitro*
h) transforming explants from double haploid plantlets or sprouts with *Agrobacterium tumefaciens* carrying at least one vector comprising a nucleotide sequence encoding a nucleotide sequence encoding the heterologous gene product under a seed specific promoter;
i) regenerating the transformed explants to transgenic doubled haploid plants;
j) determining copy number of the nucleotide sequence encoding the heterologous gene product in several doubled haploid plants;
k) selecting lines with preferred copy numbers, and
l) allowing the selected lines to produce flowers and crossing the lines, thereby receiving next generation with a increased copy number and desired level of production of the heterologic gene product.

11. The method of claim 10, wherein the haploid cell culture is *Camelina sativa* microspore culture.

12. The method of claim 11, wherein the seed specific promoter is napin - promoter.

13. The method of claim 12, wherein the heterologous gene product is selected from the group consisting of lauric acid-acyl carrier protein, hydrophobin, tropoelastin, an antibody, and a growth factor.

14. A transgenic doubled haploid seed of *Camelina sativa* expressing a heterologous gene product.

15. A double haploid dicotyledonous clonal-like production system comprising:
an isogenic doubled haploid plant line for production of seeds;
wherein the seeds are germinated and grown to flowering plants , the flowering plants are transformed with Agrobacterium -mediated flowerdip transformation to express one or more heterologous gene products , and the resulting transgenic plants are grown and allowed to produce seeds expressing the heterologous gene product.


SEQUENCE LISTING

<110>  Locusia OY

<120>  A method for protein production in  doubled haploid plants

<130>  1081 EP

<160>  26

<170>  PatentIn version 3.5

<210>  1
<211>  1470
<212>  DNA
<213>  Phaseolus vulgaris

<220>
<221>  promoter
<222>  (1)..(1470)
<223>  phaseolin promoter

<400>  1
aattcattgt actcccagta tcattatagt gaaagttttg gctctctcgc cggtggtttt      60

ttacctctat ttaaaggggt tttccaccta aaaattctgg tatcattctc actttacttg     120

ttactttaat ttctcataat ctttggttga aattatcacg cttccgcaca cgatatccct     180

acaaatttat tatttgttaa acattttcaa accgcataaa attttatgaa gtcccgtcta     240

tctttaatgt agtctaacat tttcatattg aaatatataa tttacttaat tttagcgttg     300

gtagaaagca taatgattta ttcttattct tcttcatata aatgtttaat atacaatata     360

aacaaattct ttaccttaag aaggatttcc cattttatat tttaaaaata tatttatcaa     420

atatttttca accacgtaaa tctcataata ataagttgtt tcaaaagtaa taaaatttaa     480

ctccataatt tttttattcg actgatctta aagcaacacc cagtgacaca actagccatt     540

tttttctttg aataaaaaaa tccaattatc attgtatttt ttttatacaa tgaaaatttc     600

accaaacaat gatttgtggt atttctgaag caagtcatgt tatgcaaaat tctataattc     660

ccatttgaca ctacggaagt aactgaagat ctgcttttac atgcgagaca catcttctaa     720

agtaatttta ataatagtta ctatattcaa gatttcatat atcaaatact caatattact     780

tctaaaaaat taattagata taattaaaat attacttttt taattttaag tttaattgtt     840

gaatttgtga ctattgattt attattctac tatgtttaaa ttgttttata gatagtttaa     900

agtaaatata agtaatgtag tagagtgtta gagtgttacc ctaaaccata aactataaga     960

tttatggtgg actaattttc atatatttct tattgctttt accttttctt ggtatgtaag    1020

tccgtaacta gaattactgt gggttgccat ggcactctgt ggtcttttgg ttcatgcatg    1080

gatgcttgcg caagaaaaag acaaagaaca aagaaaaaag acaaaacaga gagacaaaac    1140

gcaatcacac aaccaactca aattagtcac tggctgatca agatcgccgc gtccatgtat    1200

gtctaaatgc catgcaaagc aacacgtgct taacatgcac tttaaatggc tcacccatct 1260

caacccacac acaaacacat tgcctttttc ttcatcatca ccacaaccac ctgtatatat 1320

tcattctctt ccgccacctc aatttcttca cttcaacaca cgtcaacctg catatgcgtg 1380

tcatcccatg cccaaatctc catgcatgtt ccaaccacct tctctcttat ataataccta 1440

taaatacctc taatatcact cacttctttc 1470

<210> 2
<211> 654
<212> DNA
<213> artificial sequence

<220>
<223> chemically synthesized

<220>
<221> misc_feature
<223> sequence encoding Nicotiana tabacum leader peptide and
      Scizophyllum commune SC3 sequence

<400> 2
atgggattcg tccttttcag ccagcttcct tctttcctct tggtgtctac cctcctcttg 60

ttcctcgtga tctctcactc ttgcagggcc atgaacatcc agaccagagg taagaagatt 120

atctacggaa gcctccctag gtacaagtct ccatctcctg ctcacagctt ctctagcaga 180

actcctcagt tcaactaccc tgctctccct agcttcgcta tccttcctta caacctcctc 240

gctatgttcg ctagactccc tgttgttttc ctctacgctt tcgtggcttt cggtgctctt 300

gttgctgctc tccctggtgg acatcctgga actactactc ctcctgttac caccactgtt 360

accgttacta cccctccatc taccactact atcgctgctg gtggaacttg cactaccgga 420

tctttgtctt gctgcaacca ggtgcagtct gcttcttctt ctcctgttac tgctctcctt 480

ggactcctcg gaatcgttct ctctgatctc aacgttctcg tgggaatctc ttgctctcct 540

ctcaccgtta tcggagttgg aggatctgga tgttctgctc agactgtttg ctgcgagaac 600

acccagttca acggactcat caacatcgga tgcaccccaa tcaacatcct ctga 654

<210> 3
<211> 217
<212> PRT
<213> artificial sequence

<220>
<223> chemically synthesized

<220>
<221> PEPTIDE
<222> (1)..(217)
<223> Nicotiana tabacum leader peptide and Scizophyllum commune SC3

amino acid sequence

<400> 3

Met Gly Phe Val Leu Phe Ser Gln Leu Pro Ser Phe Leu Leu Val Ser
1               5                   10                  15

Thr Leu Leu Leu Phe Leu Val Ile Ser His Ser Cys Arg Ala Met Asn
            20                  25                  30

Ile Gln Thr Arg Gly Lys Lys Ile Ile Tyr Gly Ser Leu Pro Arg Tyr
        35                  40                  45

Lys Ser Pro Ser Pro Ala His Ser Phe Ser Ser Arg Thr Pro Gln Phe
    50                  55                  60

Asn Tyr Pro Ala Leu Pro Ser Phe Ala Ile Leu Pro Tyr Asn Leu Leu
65                  70                  75                  80

Ala Met Phe Ala Arg Leu Pro Val Val Phe Leu Tyr Ala Phe Val Ala
                85                  90                  95

Phe Gly Ala Leu Val Ala Ala Leu Pro Gly Gly His Pro Gly Thr Thr
            100                 105                 110

Thr Pro Pro Val Thr Thr Thr Val Thr Val Thr Thr Pro Pro Ser Thr
        115                 120                 125

Thr Thr Ile Ala Ala Gly Gly Thr Cys Thr Thr Gly Ser Leu Ser Cys
    130                 135                 140

Cys Asn Gln Val Gln Ser Ala Ser Ser Ser Pro Val Thr Ala Leu Leu
145                 150                 155                 160

Gly Leu Leu Gly Ile Val Leu Ser Asp Leu Asn Val Leu Val Gly Ile
                165                 170                 175

Ser Cys Ser Pro Leu Thr Val Ile Gly Val Gly Gly Ser Gly Cys Ser
            180                 185                 190

Ala Gln Thr Val Cys Cys Glu Asn Thr Gln Phe Asn Gly Leu Ile Asn
        195                 200                 205

Ile Gly Cys Thr Pro Ile Asn Ile Leu
    210                 215

<210> 4
<211> 1280
<212> DNA

<213>  artificial sequence

<220>
<223>  chemically synthesized


<220>
<221>  3'UTR
<222>  (1)..(1280)
<223>  Arcelin 3'UTR

<400>  4
actcccaaaa ccaccttccc tgtgacagtt aaaccctgct tatacctttc ctcctaataa      60

tgttcatctg tcacacaaac taaaataaat aaaatgggag caataaataa aatgggagct     120

catatattta caccatttac actgtctatt attcaccatg ccaattatta cttcataatt     180

ttaaaattat gtcattttta aaaattgctt aatgatggaa aggattatta taagttaaaa     240

gtataacata gataaactaa ccacaaaaca aatcaatata aactaactta ctctcccatc     300

taatttttat ttaaatttct ttacacttct cttccatttc tatttctaca acattattta     360

acatttttat tgtatttttc ttactttcta actctattca tttcaaaaat caatatatgt     420

ttatcaccac ctctctaaaa aaaactttac aatcattggt ccagaaaagt taaatcacga     480

gatggtcatt ttagcattaa aacaacgatt cttgtatcac tatttttcag catgtagtcc     540

attctcttca aacaaagaca gcggctatat aatcgttgtg ttatattcag tctaaaacaa     600

ttgttatggt aaaagtcgtc attttacgcc tttttaaaag atataaaatg acagttatgg     660

ttaaaagtca tcatgttaga tcctccttaa agatataaaa tgacagtttt ggataaaaag     720

tggtcatttt atacgctctt gaaagatata aaacgacggt tatggtaaaa gctgccattt     780

taaatgaaat atttttgttt tagttcattt tgtttaatgc taatcccatt taaattgact     840

tgtacaatta aaactcaccc acccagatac aatataaact aacttactct cacagctaag     900

ttttatttaa atttctttac acttcttttc catttctatt tctatgacat taactaacat     960

ttttctcgta attttttttc ttattttcta actctatcca tttcaaatcg atatatgttt    1020

atcaccacca ctttaaaaag aaaatttaca atttctcgtg caaaaaagct aaatcatgac    1080

cgtcatttta gcattaaaac aacgattctt gtatcgttgt ttttcagcat gtagtccatt    1140

cttttcaagc aaagacaaca gctatataat catcgtgtta tattcagtct aaaacaacag    1200

taatgataaa agtcatcatt ttaggccttt ctgaaatata tagaacgaca ttcatggtaa    1260

aaaatcgtca ttttagatcc                                                1280


<210>  5
<211>  2037
<212>  DNA
<213>  artificial sequence

<220>

<223>  chemically synthesized

<220>
<221>  misc_feature
<222>  (1)..(2037)
<223>  synthetic tropoelastin gene

<400>  5
tgcgcaactt tcgccctctg cttcctcctc accaacgccg gaggtgtacc aggagccatt      60

cctggtggag ttcctggagg tgtattttat ccaggagctg gtcttggagc acttggagga     120

ggcgcactag gtccaggtgg aaaacctctt aaaccagttc ctggaggact tgcgggtgct     180

ggtcttggag caggtctcgg tgcattccca gcagttacat ttccaggagc tctagtacct     240

ggtggagtag cagacgctgc tgcagcttat aaagctgcta aggctggtgc tggtcttggt     300

ggagtcccag gagttggtgg attaggagta tctgcagctc catctgtacc aggtgcagta     360

gttcctcaac ctggagcagg agttaaacct ggaaaagtac caggtgtagg tctaccagga     420

gtatacccag gtggtgtact accaggagct agattcccag gtgtaggagt gctacctgga     480

gttccaactg gtgcaggagt taagcctaag gctccaggtg taggtggagc ttttgctgga     540

attcctggag ttggaccatt tggtggtcca caaccaggag ttccactagg ataccctatt     600

aaagccccca aactacctgg tggatatgga ctaccataca caacagggaa actaccatat     660

ggttatggtc ctggaggagt agctggtgca gctggaaaag ctggttaccc aacaggtaca     720

ggtgttggcc cacaagcagc agctgcagcg gcagctaaag cagcggcaaa gttcggtgct     780

ggagcagccg gagtcctccc aggtgttgga ggtgctggtg ttcctggagt gccaggagca     840

attcctggaa ttggaggtat cgcaggtgtt ggaactccag ctgccgctgc agctgcggca     900

gcagcagcta aagcagccaa gtatggagct gctgcaggat tagtacctgg tggaccaggc     960

tttggaccag gagtagttgg tgttccagga gctggagttc caggtgttgg tgtcccagga    1020

gctggtattc cagtcgttcc tggtgctggg attccaggtg ctgcggttcc aggtgttgtg    1080

tcaccagaag cagctgctaa ggcagctgca aaggcagcta aatacggagc aagacccgga    1140

gttggagttg gaggcattcc tacttacgga gttggagctg gaggctttcc aggctttggt    1200

gttggagttg gaggaatccc tggagtggca ggtgttcctg gtgttggagg tgttccagga    1260

gttggaggtg tcccaggagt tggtatttcc cccgaagctc aagcagcagc tgctgccaaa    1320

gctgctaagt acggtttagt ccctggtgtt ggagtagctc ctggagttgg tgtagctcct    1380

ggtgtcggtg tggctccagg agttggattg gctccaggag ttggtgtagc tccaggagta    1440

ggtgtagctc ctggcgttgg agtagctcca ggcattggac ctggtggagt tgcagctgca    1500

gcaaaatccg ctgccaaggt agctgctaaa gcacaactta gagctgcagc tgggcttggt    1560

gctggaatcc ctggacttgg agttggtgtt ggcgttccag gacttggagt tggtgctggt    1620

gttcctggac ttggagttgg tgctggtgtt cctggtttcg gagcagtacc tggagcccta 1680

gctgcagcta aagcagccaa atatggagca gcagtacctg gtgttcttgg aggtctcgga 1740

gctctcggtg gagtaggcat tccaggaggt gtagttggag ctggaccagc agctgcagct 1800

gccgcagcta aagctgctgc aaaagccgcg caatttggtc tagtaggagc tgctggacta 1860

ggaggactag gagtcggagg tcttggagtt ccaggtgttg gaggccttgg aggtatacct 1920

ccagctgcag ctgctaaagc agctaaatac ggagtagcag caagaccagg tttcggattg 1980

tctccaattt tcccaggtgg tgcctgccta gggaaagctt gtggcagaaa gagaaaa 2037

<210> 6
<211> 679
<212> PRT
<213> artificial sequence

<220>
<223> chemically synthesized


<220>
<221> PEPTIDE
<222> (1)..(679)
<223> 2S2 SP-Tropoelastin amino acid sequence

<400> 6

Cys Ala Thr Phe Ala Leu Cys Phe Leu Leu Thr Asn Ala Gly Gly Val
1               5                   10                  15


Pro Gly Ala Ile Pro Gly Gly Val Pro Gly Gly Val Phe Tyr Pro Gly
            20                  25                  30


Ala Gly Leu Gly Ala Leu Gly Gly Gly Ala Leu Gly Pro Gly Gly Lys
        35                  40                  45


Pro Leu Lys Pro Val Pro Gly Gly Leu Ala Gly Ala Gly Leu Gly Ala
    50                  55                  60


Gly Leu Gly Ala Phe Pro Ala Val Thr Phe Pro Gly Ala Leu Val Pro
65                  70                  75                  80


Gly Gly Val Ala Asp Ala Ala Ala Ala Tyr Lys Ala Ala Lys Ala Gly
                85                  90                  95


Ala Gly Leu Gly Gly Val Pro Gly Val Gly Gly Leu Gly Val Ser Ala
            100                 105                 110


Ala Pro Ser Val Pro Gly Ala Val Val Pro Gln Pro Gly Ala Gly Val
        115                 120                 125

Lys Pro Gly Lys Val Pro Gly Val Gly Leu Pro Gly Val Tyr Pro Gly
    130                 135                 140

Gly Val Leu Pro Gly Ala Arg Phe Pro Gly Val Gly Val Leu Pro Gly
145                 150                 155                 160

Val Pro Thr Gly Ala Gly Val Lys Pro Lys Ala Pro Gly Val Gly Gly
                165                 170                 175

Ala Phe Ala Gly Ile Pro Gly Val Gly Pro Phe Gly Gly Pro Gln Pro
            180                 185                 190

Gly Val Pro Leu Gly Tyr Pro Ile Lys Ala Pro Lys Leu Pro Gly Gly
        195                 200                 205

Tyr Gly Leu Pro Tyr Thr Thr Gly Lys Leu Pro Tyr Gly Tyr Gly Pro
    210                 215                 220

Gly Gly Val Ala Gly Ala Ala Gly Lys Ala Gly Tyr Pro Thr Gly Thr
225                 230                 235                 240

Gly Val Gly Pro Gln Ala Ala Ala Ala Ala Ala Ala Lys Ala Ala Ala
                245                 250                 255

Lys Phe Gly Ala Gly Ala Ala Gly Val Leu Pro Gly Val Gly Gly Ala
            260                 265                 270

Gly Val Pro Gly Val Pro Gly Ala Ile Pro Gly Ile Gly Gly Ile Ala
        275                 280                 285

Gly Val Gly Thr Pro Ala Ala Ala Ala Ala Ala Ala Ala Ala Ala Lys
    290                 295                 300

Ala Ala Lys Tyr Gly Ala Ala Ala Gly Leu Val Pro Gly Gly Pro Gly
305                 310                 315                 320

Phe Gly Pro Gly Val Val Gly Val Pro Gly Ala Gly Val Pro Gly Val
                325                 330                 335

Gly Val Pro Gly Ala Gly Ile Pro Val Val Pro Gly Ala Gly Ile Pro
            340                 345                 350

Gly Ala Ala Val Pro Gly Val Val Ser Pro Glu Ala Ala Ala Lys Ala
        355                 360                 365

Ala Ala Lys Ala Ala Lys Tyr Gly Ala Arg Pro Gly Val Gly Val Gly
    370                 375                 380

Gly Ile Pro Thr Tyr Gly Val Gly Ala Gly Gly Phe Pro Gly Phe Gly
385                 390                 395                 400

Val Gly Val Gly Gly Ile Pro Gly Val Ala Gly Val Pro Gly Val Gly
                405                 410                 415

Gly Val Pro Gly Val Gly Gly Val Pro Gly Val Gly Ile Ser Pro Glu
            420                 425                 430

Ala Gln Ala Ala Ala Ala Ala Lys Ala Ala Lys Tyr Gly Leu Val Pro
        435                 440                 445

Gly Val Gly Val Ala Pro Gly Val Gly Val Ala Pro Gly Val Gly Val
    450                 455                 460

Ala Pro Gly Val Gly Leu Ala Pro Gly Val Gly Val Ala Pro Gly Val
465                 470                 475                 480

Gly Val Ala Pro Gly Val Gly Val Ala Pro Gly Ile Gly Pro Gly Gly
                485                 490                 495

Val Ala Ala Ala Ala Lys Ser Ala Ala Lys Val Ala Ala Lys Ala Gln
            500                 505                 510

Leu Arg Ala Ala Ala Gly Leu Gly Ala Gly Ile Pro Gly Leu Gly Val
        515                 520                 525

Gly Val Gly Val Pro Gly Leu Gly Val Gly Ala Gly Val Pro Gly Leu
    530                 535                 540

Gly Val Gly Ala Gly Val Pro Gly Phe Gly Ala Val Pro Gly Ala Leu
545                 550                 555                 560

Ala Ala Ala Lys Ala Ala Lys Tyr Gly Ala Ala Val Pro Gly Val Leu
                565                 570                 575

Gly Gly Leu Gly Ala Leu Gly Gly Val Gly Ile Pro Gly Gly Val Val
            580                 585                 590

Gly Ala Gly Pro Ala Ala Ala Ala Ala Ala Ala Lys Ala Ala Ala Lys
        595                 600                 605

Ala Ala Gln Phe Gly Leu Val Gly Ala Ala Gly Leu Gly Gly Leu Gly
    610                 615                 620

Val Gly Gly Leu Gly Val Pro Gly Val Gly Gly Leu Gly Gly Ile Pro
625                 630                 635                 640

Pro Ala Ala Ala Ala Lys Ala Ala Lys Tyr Gly Val Ala Ala Arg Pro
                645                 650                 655

Gly Phe Gly Leu Ser Pro Ile Phe Pro Gly Gly Ala Cys Leu Gly Lys
            660                 665                 670

Ala Cys Gly Arg Lys Arg Lys
        675

<210>  7
<211>  1145
<212>  DNA
<213>  Brassica napus

<220>
<221>  promoter
<222>  (1)..(1145)
<223>  pNap promoter

<400>  7
aagctttctt catcggtgat tgattccttt aaagacttat gtttcttatc ttgcttctga      60

ggcaagtatt cagttaccag ttaccactta tattctggac tttctgactg catcctcatt     120

tttccaacat tttaaatttc actattggct gaatgcttct tctttgagga agaaacaatt     180

cagatggcag aaatgtatca accaatgcat atatacaaat gtacctcttg ttctcaaaac     240

atctatcgga tggttccatt tgctttgtca tccaattagt gactacttta tattattcac     300

tcctctttat tactattttc atgcgaggtt gccatgtaca ttatatttgt aaggattgac     360

gctattgagc gtttttcttc aatttctttt attttagaca tgggtatgaa atgtgtgtta     420

gagttgggtt gaatgagata tacgttcaag tgaagtggca taccgttctc gagtaaggat     480

gacctaccca ttcttgagac aaatgttaca ttttagtatc agagtaaaat gtgtacctat     540

aactcaaatt cgattgacat gtatccattc aacataaaat taaaccagcc tgcacctgca     600

tccacatttc aagtattttc aaaccgttcg gctcctatcc accgggtgta acaagacgga     660

ttccgaattt ggaagatttt gactcaaatt cccaatttat attgaccgtg actaaatcaa     720

ctttaacttc tataattctg attaagctcc caatttatat tcccaacggc actacctcca     780

aaatttatag actctcatcc ccttttaaac caacttagta aacgtttttt tttttaattt     840

tatgaagtta agtttttacc ttgtttttaa aaagaatcgt tcataagatg ccatgccaga     900

acattagcta cacgttacac atagcatgca gccgcggaga attgtttttc ttcgccactt     960

gtcactccct tcaaacacct aagagcttct ctctcacagc acacacatac aatcacatgc    1020

gtgcatgcat tattacacgt gatcgccatg caaatctcct ttatagccta taaattaact    1080

catccgcttc actctttact caaaccaaaa ctcatcaata caaacaagat taaaaacata    1140

cacga                                                              1145

<210>  8
<211>  1432
<212>  DNA
<213>  artificial sequence

<220>
<223>  chemcially synthesized

<220>
<221>  misc_feature
<222>  (1)..(1432)
<223>  UcFatB1- UcFatB1-3'UTR with restriction sites

<400>  8
gaagacacaa tggccaccac ctctttagct tccgctttct gctcgatgaa agctgtaatg      60

ttggctcgtg atggccgggg catgaaaccc aggagcagtg atttgcagct gagggcggga     120

aatgcgccaa cctctttgaa gatgatcaat gggaccaagt tcagttacac ggagagcttg     180

aaaaggttgc ctgactggag catgctcttt gcagtgatca caaccatctt ttcggctgct     240

gagaagcagt ggaccaatct agagtggaag ccgaagccga agctacccca gttgcttgat     300

gaccattttg gactgcatgg gttagttttc aggcgcacct ttgccatcag atcttatgag     360

gtgggacctg accgctccac atctatactg gctgttatga atcacatgca ggaggctaca     420

cttaatcatg cgaagagtgt gggaattcta ggagatggat tcgggacgac gctagagatg     480

agtaagagag atctgatgtg ggttgtgaga cgcacgcatg ttgctgtgga acggtaccct     540

acttggggtg atactgtaga agtagagtgc tggattggtg catctggaaa taatggcatg     600

cgacgtgatt tccttgtccg ggactgcaaa acaggcgaaa ttcttacaag atgtaccagc     660

ctttcggtgc tgatgaatac aaggacaagg aggttgtcca caatccctga cgaagttaga     720

ggggagatag ggcctgcatt cattgataat gtggctgtca aggacgatga aattaagaaa     780

ctacagaagc tcaatgacag cactgcagat tacatccaag gaggtttgac tcctcgatgg     840

aatgatttgg atgtcaatca gcatgtgaac aacctcaaat acgttgcctg ggtttttgag     900

accgtcccag actccatctt tgagagtcat catatttcca gcttcactct tgaatacagg     960

agagagtgca cgagggatag cgtgctgcgg tccctgacca ctgtctctgg tggctcgtcg    1020

gaggctgggt tagtgtgcga tcacttgctc cagcttgaag gtgggtctga ggtattgagg    1080

gcaagaacag agtggaggcc taagcttacc gatagtttca gagggattag tgtgataccc    1140

gcagaaccga gggtgtaact aatgaaagaa gcatctgttg aagtttctcc catgctgttc    1200

gtgaggatac tttttagaag ctgcagtttg cattgcttgt gcagaatcat ggtctgtggt    1260

tttagatgta tataaaaaat agtcctgtag tcatgaaact taatatcaga aaaataactc    1320

aatgggtcaa ggttatcgaa gtagtcattt aagctttgaa atatgttttg tattcctcgg 1380

cttaatctgt aagctctttc tcttgcaata aagttcgcct ttcaatgggc cc 1432

<210> 9
<211> 63
<212> DNA
<213> Arabidopsis thaliana

<220>
<221> sig_peptide
<222> (1)..(63)
<223> seed storage albumin 2 signal peptide (2S2 SP)

<400> 9
atggcaaaca agctcttcct cgtctgcgca actttcgccc tctgcttcct cctcaccaac 60

gcc 63

<210> 10
<211> 57
<212> DNA
<213> artificial sequence

<220>
<223> chemcially synthesized

<220>
<221> misc_feature
<222> (1)..(57)
<223> oIL prmer

<400> 10
gttggaaata cataatctag acgtttaaac ccgggaattc attgtactcc cagtatc 57

<210> 11
<211> 42
<212> DNA
<213> artificial sequence

<220>
<223> chemically synthesized

<220>
<221> misc_feature
<222> (1)..(42)
<223> o13 primer

<400> 11
cccatgatca tgcattcaga aagaagtgag tgatattaga gg 42

<210> 12
<211> 20
<212> DNA
<213> artificial sequence

<220>
<223>  chemically synthesized


<220>
<221>  misc_feature
<222>  (1)..(20)
<223>  o5 primer

<400>  12
actcccaaaa ccaccttccc                                                    20


<210>  13
<211>  53
<212>  DNA
<213>  artificial sequence

<220>
<223>  chemcially synthesized


<220>
<221>  misc_feature
<222>  (1)..(53)

<400>  13
gactggacag ctgcggaccg ggcccggatc taaaatgacg attttttacc atg               53


<210>  14
<211>  21
<212>  DNA
<213>  artificial sequence

<220>
<223>  chemically synthesized


<220>
<221>  misc_feature
<222>  (1)..(21)
<223>  o115 primer

<400>  14
ctgcgcaact ttcgccctct g                                                  21


<210>  15
<211>  25
<212>  DNA
<213>  artificial sequence

<220>
<223>  chemically synthesized


<220>
<221>  misc_feature
<222>  (1)..(25)
<223>  o117 primer

<400>  15

ttttctcttt ctgccacaag ctttc                                          25

<210>  16
<211>  67
<212>  DNA
<213>  artificial sequence

<220>
<223>  chemically synthesized

<220>
<221>  misc_feature
<222>  (1)..(67)
<223>  o116 primer

<400>  16
gaaagcttgt ggcagaaaga gaaaacatca ccatcaccat cacaaggatg agctttgaca      60

ctcccaa                                                                67

<210>  17
<211>  55
<212>  DNA
<213>  artificial sequence

<220>
<223>  chemcially synthesized

<220>
<221>  misc_feature
<222>  (1)..(55)
<223>  o118 primer

<400>  17
agcagggttt aactgtcaca gggaaggtgg ttttgggagt gtcaaagctc atcct           55

<210>  18
<211>  50
<212>  DNA
<213>  artificial sequence

<220>
<223>  chemically synthesized

<220>
<221>  misc_feature
<222>  (1)..(50)
<223>  P-Bn-napA-F primer

<400>  18
cacacccggg aagctttctt catcggtgat tgattccttt aaagacttat                 50

<210>  19
<211>  36
<212>  DNA
<213>  artificial sequence

<220>
<223>  chemcially synthesized


<220>
<221>  misc_feature
<222>  (1)..(36)
<223>  P-Bn-napA-R  primer

<400>  19
cattcgtgta tgtttttaat cttgtttgta ttgatg                                36


<210>  20
<211>  723
<212>  DNA
<213>  artificial sequence

<220>
<223>  chemically synthesized


<220>
<221>  misc_feature
<222>  (1)..(723)
<223>  synthetic gene

<400>  20
caggttaagc tccaacaatc tggacctgag cttaagaaac ctggtgagac tgtgaagatc      60

agctgcaagg cttctggata caccttcacc gactacggaa tgaactgggt taagcaggct     120

cctggaaaag gacttaagtg gatgggatgg atcaacacct acactggtga gcctacctac     180

gctgatgatt tcaagggaag gttcgctttc agcctcgaga cttctgcttc taccgcttac     240

ctccagatca acaacctcaa gaacgaggac accgccacct atttctgcgc tagaaaggat     300

ctcctccgtt acttcgatta ctggggacag ggaactaccg ttaccgtttc ttctggtggt     360

ggtggaagcg gaggtggtgg atcaggtggt ggtggttctg atatcgagct tacccagtct     420

ccttctagcc tctctgcttc tctcggagga aaggttacca tcacttgcaa ggctagccag     480

gacatcaaca agtacattgc ctggtatcag cacaagcctg gaaagggtcc tagatctgct     540

cacacccttc acatctacat ccagcctgga atcccttcta ggttctctgg atctggaagc     600

ggaagggact actctttcag catctctaac ctcgagcctg aggatatcgc tacctactac     660

tgcctccact acgataacct tcacaccttc ggaggtggaa ctaagctcga acttaagagg     720

gct                                                                   723


<210>  21
<211>  361
<212>  PRT
<213>  artificial sequence

<220>
<223>  chemcially synthesized

<400>  21

Gln Val Lys Leu Gln Gln Ser Gly Pro Glu Leu Lys Lys Pro Gly Glu
1               5                   10                  15

Thr Val Lys Ile Ser Cys Lys Ala Ser Gly Tyr Thr Phe Thr Asp Tyr
            20                  25                  30

Gly Met Asn Trp Val Lys Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Lys Asp Leu Leu Arg Tyr Phe Asp Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Glu Leu Thr Gln Ser Pro Ser Ser Leu
    130                 135                 140

Ser Ala Ser Leu Gly Gly Lys Val Thr Ile Thr Cys Lys Ala Ser Gln
145                 150                 155                 160

Asp Ile Asn Lys Tyr Ile Ala Trp Tyr Gln His Lys Pro Gly Lys Gly
                165                 170                 175

Pro Arg Ser Ala His Thr Leu His Ile Tyr Ile Gln Pro Gly Ile Pro
            180                 185                 190

Ser Arg Phe Ser Gly Ser Gly Ser Gly Arg Asp Tyr Ser Phe Ser Ile
        195                 200                 205

Ser Asn Leu Glu Pro Glu Asp Ile Ala Thr Tyr Tyr Cys Leu His Tyr
    210                 215                 220

Asp Asn Leu His Thr Phe Gly Gly Gly Thr Lys Leu Glu Leu Lys Arg
225                 230                 235                 240

Ala Ile Asp Gly Arg Ala Ala Ala Ala Ala Ala Ala Gly Gly Ala Gly
                245                 250                 255

Gln Gly Gly Tyr Gly Gly Leu Gly Ser Gln Gly Ala Gly Arg Gly Gly
            260                 265                 270

Gln Gly Ala Gly Ile Asp Gly Arg Gln Pro Leu Glu Asp Arg Ser Asn
        275                 280                 285

Gly Asn Gly Asn Val Cys Pro Pro Gly Leu Phe Ser Asn Pro Gln Cys
    290                 295                 300

Cys Ala Thr Gln Val Leu Gly Leu Ile Gly Leu Asp Cys Lys Val Pro
305                 310                 315                 320

Ser Gln Asn Val Tyr Asp Gly Thr Asp Phe Arg Asn Val Cys Ala Lys
                325                 330                 335

Thr Gly Ala Gln Pro Leu Cys Cys Val Ala Pro Val Ala Gly Gln Ala
            340                 345                 350

Leu Leu Cys Gln Thr Ala Val Gly Ala
        355                 360

<210>  22
<211>  23
<212>  DNA
<213>  artificial sequence

<220>
<223>  chemcially synthesized

<220>
<221>  misc_feature
<222>  (1)..(23)
<223>  cCEA-R primer

<400>  22
agccctctta agttcgagct tag                                              23

<210>  23
<211>  65
<212>  DNA
<213>  artificial sequence

<220>
<223>  chemically synthesized

<220>
<221>  misc_feature
<222>  (1)..(65)
<223>  aCEA-His-F primer

<400>  23
ctaagctcga acttaagagg gctcatcacc atcaccatca caaggatgag ctttgacact      60

cccaa                                                                  65


<210>  24
<211>  35
<212>  DNA
<213>  artificial sequence

<220>
<223>  chemically synthesized


<220>
<221>  misc_feature
<222>  (1)..(35)
<223>  o11 primer

<400>  24
tgaatgcatg atcatgggat tcgtcctttt cagcc                                 35


<210>  25
<211>  41
<212>  DNA
<213>  artificial sequence

<220>
<223>  chemically synthesized


<220>
<221>  misc_feature
<222>  (1)..(41)
<223>  o12 primer

<400>  25
ggaaggtggt tttgggagtt cagaggatgt tgataggggt g                          41


<210>  26
<211>  13
<212>  DNA
<213>  artificial sequence

<220>
<223>  chemically synthesized


<220>
<221>  5'UTR
<222>  (1)..(13)
<223>  arcelin 5'UTR

<400>  26
tgaatgcatg atc                                                         13